# EUROPÄISCHE PATENTSCHRIFT

(11) **EP 2 456 465 B1**
(45) Veröffentlichungstag und Bekanntmachung des Hinweises auf die Patenterteilung: **06.09.2023**
(21) Anmeldenummer: 10737857.2
(22) Anmeldetag: 21.07.2010
(51) Int. Cl.: A61K 41/00, A61K 31/357, C01B 13/02, A61P 35/00

(54) **ENDOPEROXIDHALTIGES MATERIALSYSTEM MIT ANPASSUNG DES ZERFALLS UND ANWENDUNGEN**
MATERIAL SYSTEM COMPRISING ENDOPEROXIDE WITH ADAPTATION OF ITS DECOMPOSITION, AND ITS APPLICATIONS
SYSTÈME DE MATÉRIAU CONTENANT DE L'ENDOPEROXYDE AVEC ADAPTATION DE SA DÉSINTÉGRATION, ET SES UTILISATIONS

(30) Priorität: 21.07.2009 DE 102009034279
(43) Veröffentlichungstag der Anmeldung: 30.05.2012
(73) Patentinhaber: Bogner, Udo, 93053 Regensburg (DE); Bernhardt, Günther, 84069 Schierling (DE); Knör, Günther, 4210 Gallneukirchen (AT)
(72) Erfinder: Bogner, Udo, 93053 Regensburg (DE); Bernhardt, Günther, 84069 Schierling (DE); Knör, Günther, 4210 Gallneukirchen (AT)
(74) Vertreter: Hannke Bittner & Partner mbB Regensburg
(86) Internationale Anmeldenummer: PCT/EP2010/060594
(87) Internationale Veröffentlichungsnummer: WO 2011/009903

(56) Entgegenhaltungen:
- AU-B2- 548 020
- US-A- 4 436 715
- Franz-Peter Kalz: "Untersuchungen für die Anwendung von aromatischen Endoperoxiden in Liposomen und Polymeren zur Tumortherapie", Dissertation zur Erlangung des Doktorgrades der Naturwissenschaften, 25. September 2006 (2006-09-25), Seiten 1-116, XP55003201, Regensburg, DE Gefunden im Internet: URL:http://epub.uni-regensburg.de/12310/1/ DissertationvonFranz_Peter_Kalz.pdf [gefunden am 2011-07-20]
- NICHOLAS J. TURRO ET AL: "Generation, diffusivity, and quenching of singlet oxygen in polymer matrixes investigated via chemiluminescence methods", THE JOURNAL OF PHYSICAL CHEMISTRY, Bd. 85, Nr. 20, 1. Oktober 1981 (1981-10-01), Seiten 3014-3018, XP55001917, ISSN: 0022-3654, DOI: 10.1021/j150620a034
- KAZAKOV D V ET AL: "Chemiluminescence during decomposition of 1,4-dimethylnaphthalene endoperoxide on the silica gel and alumina surface", RUSSIAN CHEMICAL BULLETIN, Bd. 56, Nr. 2, 1. Februar 2007 (2007-02-01), Seiten 205-210, XP019533475, ISSN: 1573-9171, DOI: 10.1007/S11172-007-0034-Z
- HARRY H. WASSERMAN ET AL: "Photooxidation of Methylnaphthalenes", THE JOURNAL OF ORGANIC CHEMISTRY, Bd. 70, Nr. 1, 9. Dezember 2004 (2004-12-09), Seiten 105-109, XP55001615, ISSN: 0022-3263, DOI: 10.1021/jo040228v
- O'NEILL P M ET AL: "Lewis acid catalysed rearrangements of unsaturated bicyclic [2.2.n] endoperoxides in the presence of vinyl silanes; access to novel Fenozan BO-7 analogues", TETRAHEDRON LETTERS, Bd. 46, Nr. 17, 25. April 2005 (2005-04-25), Seiten 3029-3032, XP004829123, ISSN: 0040-4039, DOI: 10.1016/J.TETLET.2005.03.022
- Q. JASON NIU ET AL: "Yields of singlet molecular oxygen from peroxyl radical termination", JOURNAL OF THE AMERICAN CHEMICAL SOCIETY, Bd. 114, Nr. 1, 1. Januar 1992 (1992-01-01), Seiten 165-172, XP55001637, ISSN: 0002-7863, DOI: 10.1021/ja00027a024
- KAORU OTSU ET AL: "An abortive apoptotic pathway induced by singlet oxygen is due to the suppression of caspase activation", BIOCHEMICAL JOURNAL, Bd. 389, Nr. 1, 1. Juli 2005 (2005-07-01), Seiten 197-206, XP55001889, ISSN: 0264-6021, DOI: 10.1042/BJ20042067
- NAGAOKA Y ET AL: "Specific inactivation of cysteine protease-type cathepsin by singlet oxygen generated from naphthalene endoperoxides", BIOCHEMICAL AND BIOPHYSICAL RESEARCH COMMUNICATIONS, Bd. 331, Nr. 1, 27. Mai 2005 (2005-05-27), Seiten 215-223, XP004858639, ISSN: 0006-291X, DOI: 10.1016/J.BBRC.2005.03.146
- SCHARFFETTER-KOCHANEK K ET AL: "Singlet oxygen induces collagenase expression in human skin fibroblasts", FEBS LETTERS, Bd. 331, Nr. 3, 4. Oktober 1993 (1993-10-04), Seiten 304-306, XP025598585, ISSN: 0014-5793, DOI: 10.1016/0014-5793(93)80357-Z [gefunden am 1993-10-04]
- DAMIR POSAVEC ET AL: "Functionalized derivatives of 1,4-dimethylnaphthalene as precursors for biomedical applications: synthesis, structures, spectroscopy and photochemical activation in the presence of dioxygen", ORGANIC & BIOMOLECULAR CHEMISTRY, vol. 10, no. 35, 20 July 2012 (2012-07-20) , pages 7062-7069, XP055286053, GB ISSN: 1477-0520, DOI: 10.1039/c2ob26236c
- CLANCY CORNELIUS J. ET AL: "Emergence of Candida auris : An International Call to Arms", CLINICAL INFECTIOUS DISEASES, vol. 64, no. 2 20 October 2016 (2016-10-20), pages 141-143, XP055755330, US ISSN: 1058-4838, DOI: 10.1093/cid/ciw696 Retrieved from the Internet: URL:https://academic.oup.com/cid/article-p df/64/2/141/8960495/ciw696.pdf

## Beschreibung

Die vorliegende Erfindung bezieht sich auf ein Verfahren zur Herstellung von Singulett-Sauerstoff durch den Zerfall von Endoperoxidmolekülen in Materialsystemen und dessen Verwendung.

Im bisherigen Stand der Technik wird Singulett-Sauerstoff bereits im pharmazeutischen Bereich, beispielsweise in der Photodynamischen Therapie (PDT), eingesetzt. Hierzu wird vor der Therapie eine photosensibilisierende Substanz, ein sog. Sensitizer, verabreicht, welcher sich in höherer Konzentration im Tumorgewebe ansammelt als im Normalgewebe. Durch Lichtbestrahlung wird der Sensitizer angeregt und kann bei Vorhandensein von Sauerstoff Singulett-Sauerstoff erzeugen, welcher lethale oxidative Schäden verursacht, wobei beispielsweise Membranlipide geschädigt werden.

Im Rahmen einer Dissertation zur Erlangung des Doktorgrades der Naturwissenschaften an der Universität Regensburg (Franz-Peter Kalz: "Untersuchungen für die Anwendung von aromatischen Endoperoxiden in Liposomen und Polymeren zur Tumortherapie", 25. September 2006), wurden verschiedene Systeme mit Endoperoxiden in Liposomen und in Polymeren durch Verwendung der Methode der PDT erzeugt. Hiermit wurde bei den ohnehin lediglich zur Ergänzung durchgeführten Untersuchungen mit Polymeren, bevorzugt die Bildung von Endoperoxidmolekülen in Polymeren durch detaillierte Messungen ermittelt, während für den Zerfall der Endoperoxidmoleküle in Polymeren nur erste Messungen erfolgten, die sich außerdem nur auf den Anfang der Zerfallskurve bezogen.

Aufgrund des Zusammenspiels von Sensitizer und Bestrahlung durch (Laser-)licht ist die PDT in ihrem Anwendungsbereich aufgrund der limitierten Eindringtiefe des therapeutischen Lichts eingeschränkt. Die PDT wird daher bevorzugt zur lokalen Behandlung oberflächlicher Karzinome auf der Haut oder endoskopisch zugänglichen Hohlorganen eingesetzt.

Daher ist es Aufgabe der vorliegenden Erfindung durch Freisetzung von Singulett-Sauerstoff (¹O₂) bestimmte biologische Objekte, insbesondere Zellen in der Tumortherapie, wie beispielsweise Krebszellen, oder resistente Keime (beispielsweise durch antimikrobielle Behandlung) abzutöten. Wegen der geringen Lebensdauer von Singulett-Sauerstoff besteht das technisch wichtigste Problem darin, dass der Abstand zwischen dem Ort der Erzeugung und dem Ort der Wirkung (also dem Zielgebiet im Innern oder an der Oberfläche der Zelle) sehr klein gehalten werden muss. Dieser Abstand hängt vom jeweiligen Medium ab, in dem der Singulett-Sauerstoff bis ins Zielgebiet diffundieren soll und wird in der Regel kleiner als 1 µ sein. Dies wird erfindungsgemäß durch ein Verfahren nach Anspruch 1 erreicht. Vorteilhafte Ausführungsformen und Weiterbildungen sind Gegenstand der Unteransprüche.

Der Grundgedanke der vorliegenden Erfindung besteht darin, dass bei der Herstellung von Singulett-Sauerstoff durch den Zerfall von Endoperoxidmolekülen mit Hilfe eines sich aus wenigstens zwei Molekülen zusammensetzenden Materialsystems, das zumindest ein eine Endoperoxidgruppe bildendes Molekül enthält, eine Anpassung eines zeitlichen Verlaufs eines Zerfalls der Endoperoxidmoleküle ermöglicht wird.

Singulett-Sauerstoff wird im Rahmen der Erfindung bevorzugt durch den thermisch aktivierten Zerfall von aromatischen Endoperoxidmolekülen erzeugt, welche in den als pharmazeutische Träger verwendeten Materialsystemen wie beispielsweise Liposomen, Polymernanopartikeln oder dünnen Polymerfilmen, eingebaut sind und in einer ausreichend großen Anzahl von Einbaulagen eine genügend gut angepasste Zerfallszeit t_{z} aufweisen. Genügend angepasst bedeutet insbesondere, dass die Endoperoxidmoleküle zum größten Teil erst dann zerfallen, wenn diese Materialsysteme in ausreichend geringem Abstand vom Zielgebiet sind, beispielsweise im Inneren der Zelle oder an deren Oberfläche, oder die Zellen befinden sich auf dem Polymerfilm. Es ist entscheidend, dass der gesamte zeitliche Verlauf der Singulett-Sauerstoff-Freisetzung, insbesondere die im Endoperoxid/Materialsystem auftretende Verteilung der Zerfallszeiten t_{z} an die Details der jeweiligen Anwendung angepasst sind, so dass die durch die Singulett-Sauerstoffmoleküle erzeugten Schäden zur Abtötung der Zelle ausreichen. Es müssen genügend Endoperoxidmoleküle in dem Materialsystem Zerfallszeiten haben, welche größer sind als die Zeit tₐ für die Aufnahme des Polymers in die Zelle (beispielsweise durch Endozytose) oder als die Zeit für die Anlagerung eines Bakteriums nach einer Operation an ein Implantat (z. B. Zahnimplantat oder künstliches Gelenk) auf dem als Schutzfilm aufgetragenen Materialsystem.

Mit Hilfe eines konfokalen Mikroskops wurden beispielsweise für Liposomen mit einem Durchmesser von 110 nm und Polymernanoteilchen mit einem Durchmesser von 140 nm in bestimmten Krebszellen bei 37°C entsprechende Zeiten tₐ mit einem Mittelwertbereich von einigen Stunden gemessen. Andererseits dürfen die Zerfallszeiten nicht zu lange sein, da die Zellen die Nanoteilchen in der Regel nach einiger Zeit wieder ausscheiden. Außerdem müssen die durch die Singulett-Sauerstoffmoleküle hervorgerufenen Schäden im Zielgebiet der Zelle in zeitlich kürzerem Abstand erzeugt werden, als sie durch Reparaturmechanismen wieder beseitigt werden können.

Der erste Schritt der Anpassung der Halbwertszeit des Zerfalls bzw. der entsprechenden Verteilung bei Auftreten von mehreren Halbwertszeiten besteht in der Festlegung einer geeigneten Kombination der Komponenten des Materialsystems.

Erfindungsgemäß erfolgt die Anpassung des zeitlichen Verlaufs des Zerfalls der Endoperoxidmoleküle durch eine physikalische Veränderung der im Materialsystem vorhandenen Funktionsstrukturen. Alle Anwendungsbeispiele, welche bereits realisiert wurden, zeigen, dass es möglich ist, durch physikalische Veränderungen dieser Funktionsstrukturen, die erfindungsgemäß jeweils aus dem Endoperoxid bildenden Molekül und seiner unmittelbaren Umgebung im Trägermaterial bestehen, Veränderungen der Barrierenhöhe(n) für den Zerfall und damit Veränderungen der Zerfallszeit(en) zu bewirken, welche zum Teil mehr als eine Größenordnung betragen, so dass sie angewendet werden können, um bevorzugt eine Anpassung des gesamten Verlaufs des Zerfalls für den gewünschten Verwendungszweck, beispielsweise in der Tumortherapie, zu bewirken.

Eine chemische Veränderung kann beispielsweise durch das Hinzufügen funktioneller Gruppen, wie beispielsweise Methylgruppen oder Hydroxylgruppen, in der Peripherie des Endoperoxid bildenden Moleküls oder im umgebenden Trägermaterial erfolgen. So ermöglicht eine polare Hydroxylgruppe die Ausbildung von chemischen Bindungen, wie beispielsweise Wasserstoff-Brückenbindungen.

Bei physikalischen Veränderungen der Funktionsstrukturen handelt es sich erfindungsgemäß um eine Veränderung der Einbaulage des Endoperoxid bildenden Moleküls im Käfig des umgebenden Trägermaterials und/oder um eine Veränderung des Käfigs bzw. der Käfigwand.

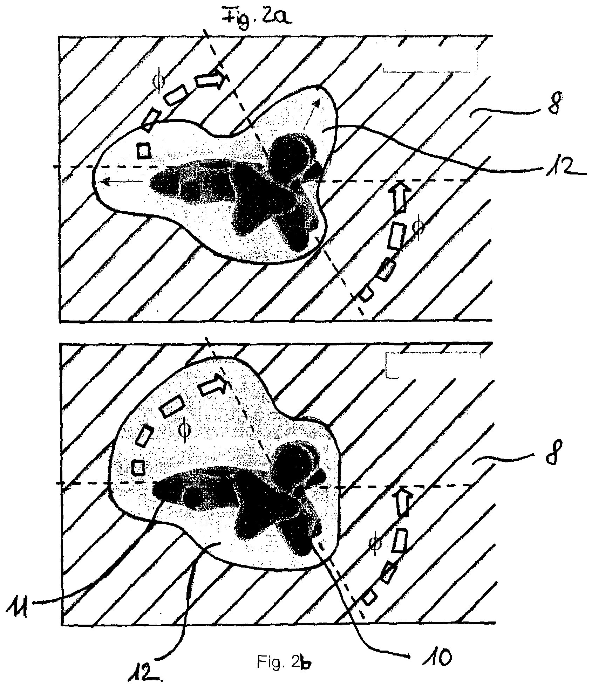

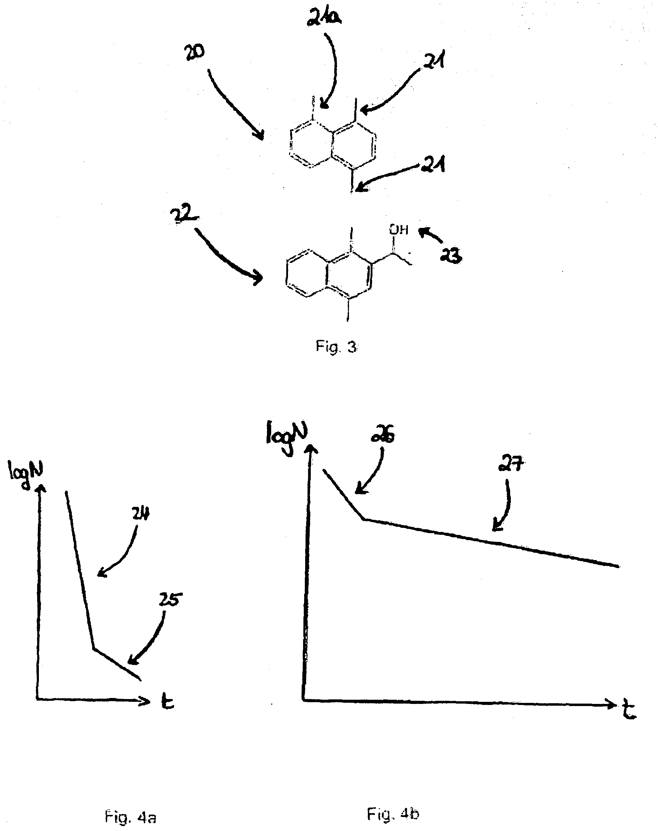

Das erfindungsgemäße Prinzip der physikalischen Veränderung einer speziellen Funktionsstruktur bestehend aus einem Endoperoxid bildenden Molekül und dem Käfig der umgebenden Polymermatrix beinhaltet folgendes Vorgehen bei der Präparation. Nach einer Prozedur der Endoperoxidbildung lässt man (bevorzugt thermisch aktiviert) einen Zerfallsprozess ablaufen, bei dem es durch Umlagerungsprozesse zu einer Veränderung der Funktionsstruktur kommt. Wird nach diesen Präparationsschritten erneut eine Endoperoxidbildung durchgeführt, so ergibt sich anschließend eine veränderte Zerfallszeit, welche auf die mit der geometrischen Änderung der Funktionsstruktur verknüpften Änderung der Barrierenhöhe für den Zerfall zurück zu führen ist. Die Details sind aus der Erklärung der Figur 2 und 4 ersichtlich.

Erfindungsgemäß stellt ein erstes oder mindestens ein weiteres Molekül des Materialsystems ein Trägermaterial dar, wobei das Trägermaterial organisch und/oder anorganisch ausgebildet ist. Das Trägermaterial weist in seinem Volumen zahlreiche Moleküle auf und verfügt über eine geeignete mikroskopische und makroskopische Struktur, wobei die Endoperoxidmoleküle auf der Oberfläche des Trägermaterials angelagert oder in seinem Inneren eingebaut sind. Das Trägermaterial ermöglicht zudem den Transport der Endoperoxid bildenden Moleküle zum Zielgebiet oder ist selbst als Zielgebiet anzusehen, wenn es z. B. als Schutzfilm auf einem Implantat aufgetragen ist, auf dem sich beispielsweise Bakterien absiedeln. Es ist denkbar als organisches Trägermaterial Liposomen, Lipoproteine (bei denen vor der Verwendung im Materialsystem Umwandlung der Doppelbindungen in Einfachbindungen durchgeführt wurde) oder auch Polymere einzusetzen.

Die Herstellung der Liposomen erfolgt hierbei beispielsweise durch Ultraschallbehandlung oder durch Extrusionstechnik. Die Polymere können beispielsweise als Polymerfilm, welcher die Trägermatrix bildet, oder auch als Nanopartikel vorliegen. Als anorganisches Trägermaterial sind beispielsweise Silicaverbindungen denkbar oder γ-Aluminiumoxid und ähnliche zur Adsorption verwendete Materialien.

Bei einer weiteren vorteilhaften Ausführungsform ist das Endoperoxid bildende Molekül des Materialsystems eine aromatische Verbindung. Vorzugsweise werden als Endoperoxid bildendes Molekül ein Naphthalinderivat, wie beispielsweise 1,4-Dimethylnaphthalin (DMN) oder dessen Derivate, eingesetzt. Bei der Reaktion von diesen Naphthalinderivaten mit Singulett-Sauerstoff entstehen Endoperoxide. Diese Reaktion ist durch einen bevorzugt thermisch aktivierten Prozess reversibel ausgebildet. Das bedeutet, dass die Endoperoxide zu einem großen Teil bei Erwärmung wieder in das zu Grunde liegende aromatische Molekül und den Singulett-Sauerstoff zerfallen, wobei die Zerfallszeiten von den Endoperoxiden bei einer bestimmten Temperatur in der Regel unterschiedlich sind. Bei Endoperoxidbildung wird die Endoperoxidbrücke an den Kohlenstoffatomen der jeweils gegenüberliegenden Stellen des Benzolrings ausgebildet, an welchem zusätzliche Substituenten, wie beispielsweise eine Methylgruppe, angeordnet sind. Bei diesem Prozess wird Singulett-Sauerstoff eingefangen und gewissermaßen "gespeichert". 1,4-Dimethylnaphthalin kann bezüglich der Toxizität als unbedenklich eingestuft werden, da es bereits in Lebensmitteln wie der Kartoffel enthalten ist, um das Keimen zu verhindern, und verwendet wurde, um Speisekartoffeln beim Einlagern haltbar zu machen.

Ferner wäre auch denkbar, bestimmte aromatische Verbindungen wie beispielsweise Lebensmittelfarbstoffe als Endoperoxid bildende Moleküle einzusetzen. Bevorzugt sind solche aromatischen Verbindungen bezüglich ihrer Toxizität als unbedenklich einzustufen.

Bei einer weiteren vorteilhaften Ausführungsform bildet die aromatische Verbindung durch Autoperoxidation Endoperoxide, d. h. die Verbindung wirkt selbst als Sensitizer (engl. selfsensitized peroxidation). Unter Autoperoxidation ist die Erzeugung und der Einfang von Singulett-Sauerstoff mit ein und demselben aromatischen Molekül zu verstehen. Dabei wird das aromatische Molekül mit Licht geeigneter Wellenlänge vom elektronischen Grundzustand in den elektronisch angeregten Singulett-Zustand überführt. Es erfolgt durch Intersystem Crossing der Übergang in den Triplett-Zustand des aromatischen Moleküls. Durch Energietransfer wird die Anregungsenergie des Triplett-Zustands auf den molekularen Sauerstoff übertragen. Dabei entsteht elektronisch angeregter Singulett-Sauerstoff, während das aromatische Molekül wieder in seinen Grundzustand übergeht. Dies ist interessant, wenn es vorteilhaft ist, dass dem Materialsystem keine zusätzliche photosensibilisierende Substanz zugegeben werden muss. Als aromatische Verbindungen für eine derartige Autoperoxidation sind beispielsweise meso-Diphenylenhelianthrene denkbar.

Bei einer weiteren bevorzugten Ausführungsform wird alternativ oder zusätzlich ein magnetisches Material im Materialsystem verwendet. Hierbei handelt es sich bevorzugt um antiferromagnetisches Material.

Die Erhöhung der Barriere im Beispiel der PVB-Polymerfilme ist bevorzugt für eine bestimmte Anwendung von Interesse. Da diese Filme bevorzugt aus völlig amorphen Polymermaterial mit einer Dicke von unter 100 nm herstellbar sind, könnte man sie zum Beschichten (coating) von magnetischen Nanopartikeln, welche bevorzugt in Form von kolloidalen Lösungen als sog. Ferrofluide vorliegen, verwenden.

In den zurzeit entwickelten Therapieverfahren mit diesen magnetischen Nanopartikeln werden diese mit einem Magnetfeld an den gewünschten Ort im Körper, beispielsweise in ein Tumorgebiet geführt. Diese magnetischen Nanopartikel können in einer Beschichtung bevorzugt auch einen Wirkstoff gezielt zum Tumor transportieren (Magnetic Drug Targeting), außerdem kann durch magnetische Wechselfelder eine lokale Hyperthermie bis maximal 46°C durchgeführt werden. Bei Beschichtung der magnetischen Nanoteilchen mit beispielsweise amorphen Polymerfilmen, die mit Endoperoxid dotiert sind, könnte dabei Singulett-Sauerstoff im Tumorgebiet freigesetzt werden. Eine Anpassung der Barrierehöhe sollte beinhalten, dass erst bei 46°C und nicht schon bei 37°C, also beim Transport der Nanoteilchen, eine Freisetzung des Singulett-Sauerstoffs erfolgt.

Diese lokale Freisetzung aus der Beschichtung eines magnetischen Nanoteilchens, das als Implantat betrachtet werden kann, lässt sich auch auf andere Implantate, z. B. auf künstliche Gelenke, oder allgemein auf medizinische Implantate übertragen. Es ist von besonderem Interesse, auf medizinischen Implantaten Beschichtungen aufzubringen (siehe z. B.: Hans Gollwitzer, Eine resorbierbare Poly-D,L-Laktid-Beschichtung zur Ausrüstung medizinischer Implantate, Dissertation, Technische Universität München, 2002).

Hierbei steht in der Regel die Freisetzung (Elution) von Antibiotika aus der Beschichtung im Vordergrund, um einen Schutz gegen Bakterien zu erzielen, da eine Bakterienkolonisation sich als infektionsbedingte Abstoßung von Implantaten auswirken kann. Bei einer Verwendung von endoperoxidhaltigen Materialen könnte man nicht nur bei Implantaten, sondern allgemein wegen der antiinfektiösen Wirkung des beim Zerfall freiwerdenden Singulett-Sauerstoffs Bakterien, Viren und Pilze abtöten.

Bezüglich der abtötenden Wirkung auf Bakterien ist es denkbar, vor allem auch Bakterien mit Resistenzen (z. B. Methicillin resistente Bakterien Staphylococcus aureus, MRSA) oder allgemein multiresistente Bakterien einzubeziehen, da die abtötende Wirkung von Singulett-Sauerstoff mit Hilfe der Photodynamischen Therapie schon nachgewiesen wurde (für einen Überblick siehe z. B. den Artikel von Mariusz Grinholc et al, Bactericidal effect of photodynamic therapy against methicillin-resistant staphylococcus aureus strain with the use of various porphyrin photo sensitizers, Acta Biochemica Polonica 54 (2007) 665-670, on-line at: www.actabp.pl, und die in diesem Artikel angegebenen Literaturstellen).

Als bester Weg zum Einsatz der Erfindung erscheint bezüglich der Behandlung bestimmter Tumore die Kombination mit der Methode des vorher erläuterten Magnetic Drug Targeting einschließlich der damit verknüpften lokalen Hyperthermie. Die wichtigsten Details dieser Methode sind zu entnehmen aus dem Übersichtsartikel von Andreas S. Ziegler, "Nanopartikel - pharmazeutische Zwerge mit Know-how", Medizinische Monatsschrift für Pharmazeuten 31 (2008), 455-468. Durch die Kombination mit dieser Methode ließe sich eine selektive Wirkung des Endoperoxidzerfalls auf den Tumor erzielen, in dem durch die lokale Temperaturerhöhung eine verstärkte Freisetzung von Singulett-Sauerstoff im Tumor erreichbar ist, da in ihm die magnetischen Nanoteilchen angereichert sind. Hierbei sollte auf der Oberfläche der magnetischen Nanoteilchen (die selbst einen möglichst kleinen Durchmesser haben sollen) eine Polymerschicht mit einer Dicke kleiner als die Diffusionslänge in diesem Polymermaterial aufgetragen sein, auf jeden Fall sollte der Gesamtdurchmesser unter 200 nm sein, damit die Teilchen leicht in der Krebszelle aufgenommen werden. Das Polymer sollte bioabbaubar und resorbierbar und biokompatibel sein. Hierbei bietet es sich an, z. B. ein Polymer zu verwenden wie es sonst für medizinische Implantate üblich ist wie z. B. das Poly-D,L-Laktid (PDLLA), das als amorphes Material im Gegensatz zum semikristallinen Poly-L-Laktid bevorzugt wird (siehe: Hans Gollwitzer, "Eine resorbierbare Poly-D,L-Laktid-Beschichtung zur Ausrüstung medizinischer Implantate", Dissertation, Technische Universität München, 2002; Seite 19).

Als Endoperoxid bildendes Molekül könnte Dimethylnaphthalin (oder ein Derivat) als Dotierung verwendet werden. Es ist zu erwarten, dass mit dem dotierten Polymer alle Eigenschaften erreicht werden, die mit dem oben beschriebenen amorphen Polyvinylbutyral (PVB) erreicht wurden, d. h. insbesondere eine Anpassung der Zerfallszeit (bei 37 °C und anschließend bei der während der Phase der Hyperthermie eingestellten Temperatur von ca. 42 °C bis 46 °C), wobei diese Anpassung mit chemischen und/oder physikalischen (siehe Figur 4) Veränderungen der Funktionsstrukturen des Polymers erfolgen kann. Bei der Anpassung des gesamten Zeitverlaufes des Zerfalls der Endoperoxidmoleküle ist es bei dieser Anwendung wichtig, dass insbesondere während des bei 37 °C stattfindenden Transportes zur Krebszelle und im Verlauf des Vorganges des Eindringens in die Krebszelle noch möglichst wenig Endoperoxidmoleküle zerfallen, wohingegen in der Krebszelle in der Phase der Hyperthermie im Magnetfeldapplikator (wegen der Erwärmung durch das magnetische Wechselfeld) für eine Behandlungszeit von ca. über einer Stunde möglichst viel Singulett-Sauerstoff freigesetzt werden soll. Hierbei ist zu beachten, dass bei erhöhter Temperatur bestimmte Reparaturmechanismen innerhalb der Krebszellen außer Kraft gesetzt und somit die zellschädigende Wirkung des Singulett-Sauerstoffs erhöht wird.

Bei einer weiteren vorteilhaften Ausführungsform wird ein Sensitizer im Materialsystem entfernbar eingesetzt. Bei Einbau der Sensitizer in das Trägermaterial (beispielsweise Liposomen oder Polymerfilme) finden in der Regel Protoporphyrinderivate, wie beispielsweise Protoporphyrin IX-Dimethylester, Verwendung, welche mit einer Hochleistungs-LED oder mit einem Laser angeregt werden. Bei allen Polymernanoteilchen wird bevorzugt Methylenblau als Sensitizer verwendet, welches der wässrigen Suspension zugesetzt wird; die Anregung erfolgt in diesen Fällen bevorzugt mit einem Diodenlaser (660nm) oder einer entsprechenden LED. Dies ist vorteilhaft, da so die Bereitstellung des Singulett-Sauerstoffs für die Endoperoxidbildung bevorzugt außerhalb des Körpers stattfindet, so dass lediglich die Endoperoxidmoleküle dem Organismus zugeführt werden. Somit erfolgt die Endoperoxidbildung bevorzugt räumlich und/oder zeitlich getrennt. Die Materialsysteme können daher beispielsweise am Tag vor der Behandlung des Patienten hergestellt oder unmittelbar vor der Behandlung durch Lichteinstrahlung, bevorzugt mit Hilfe des Sensitizers, "aktiviert" und als Salbenzusatz oder Emulsion bereitgestellt werden. Der Patient ist somit während der Behandlung bevorzugt keiner Bestrahlung ausgesetzt.

Bei einer weiteren vorteilhaften Ausführungsform geht die Endoperoxid bildende Verbindung mit mindestens einem Molekül, welches benachbart angeordnet ist und nicht Teil der Endoperoxid bildenden Verbindung ist, eine chemische Bindung beispielsweise mit dem Polymermaterial des Trägers ein. Unter chemischer Bindung sind insbesondere WasserstoffBrückenbindungen und kovalente Bindungen zu verstehen, wobei auch jegliche Art von Inter- und Intramolekularen Wechselwirkungen hinzu zählen.

Bei einer weiteren vorteilhaften Ausführungsform wird das erfindungsgemäße Verfahren - wie bereits mehrfach erwähnt - im pharmazeutischen und/oder medizinischen Bereich angewendet. Allgemein wird bei einer Therapie in der Pharmazie vor allem eine gezielte Freisetzung von Pharmazeutika angestrebt. Hierbei wird von Arzneiformen insbesondere erwartet, dass sie einen pharmakologisch aktiven Wirkstoff unter kontrollierten

Bedingungen gezielt an seinen Wirkort im Körper bringen ("Drug Targeting") und dort unter definierten Bedingungen freisetzen ("Drug Release"). Insbesondere Nanopartikel stellen hierbei eine innovative Arzneiform dar, mit denen dieses Ziel erreicht werden kann. Dabei ist der Einsatz von Liposomen ebenso denkbar wie die Anwendung von wässrigen Polymerdispersionen zur Steuerung der Wirkstofffreigabe. Ferner sind beschichtete Nanopartikel aus schnell abbaubarem Polymer denkbar. Alle Nanopartikel können speziell beschichtet sein, beispielsweise mit Tensiden, so dass sie die Blut-Hirn-Schranke überwinden. Darüber hinaus sind auch Nanoteilchen aus Ethylcellulose und PVB (Polyvinyylbutyral), vor allem im Verdauungstrakt denkbar, da Ethylcellulose ohnehin bei der Herstellung von Tabletten als Hilfsstoff verwendet wird und PVB für die Verpackung von Lebensmitteln zugelassen ist, also in den Verdauungstrakt gelangen darf.

Als weitere pharmazeutisch/medizinische Anwendung für die Behandlung der Haut (z. B. bei bestimmten Tumoren oder bei Psoriasis) ist der Einsatz von Endoperoxid haltigen Materialsystemen denkbar, die Liposomen oder Nanoteilchen in einer Salbe oder Emulsion enthalten. Auch hier ist die Anpassung des Zeitverlaufes des thermisch induzierten Zerfalls an die Details der Anwendung wichtig. Im Gegensatz zur Verwendung von Polymer(nano)teilchen und anorganischen (Nano-)Teilchen - bei denen auch die nach Fig. 4 durchgeführte physikalische Modifikation der Funktionsstrukturen zur Anpassung der Zerfallszeit in einem weiten Bereich eingesetzt werden kann, muss die Anpassung bei Liposomen durch eine Auswahl der chemischen Modifizierungen ermöglicht werden. Für das Liposomenmaterial DMPC (1,2-Dimyristol-L-α-Phosphatidylcholine), das ausgewählt wurde, da es keine Doppelbindungen enthält, erhält man z. B. mit den Endoperoxiden von DMN bzw. DMNOH als Dotierung eine Halbwertszeit (mit einem für mit Endoperoxid dotierte Liposomen typischen monoexponentiellen Zerfall) von ca. einer Stunde bzw. elf Stunden bei 37 °C.

Ferner ist denkbar, das erfindungsgemäße Verfahren auch in der Produktionstechnik einzusetzen, insbesondere wenn organische Werkstoffe verwendet werden, da Singulett-Sauerstoff sehr reaktionsfähig ist und mit vielen organischen Verbindungen reagiert (z. B. bei Oxidationsprozessen, Syntheseschritten, Veränderungen der Doppelbindungen, usw.). Auch hierbei ist zu erwarten, dass die Anpassung des Zeitverlaufs des Endoperoxidzerfalls (im Zeitbereich von Sekunden bis Minuten bei lokalisierten Temperaturen bis über 100 °C) und damit der entsprechenden Singulett-Sauerstofferzeugung an die Erfordernisse der Anwendung in bestimmten Produktionsschritten von entscheidender Bedeutung ist. Dies gilt z. B. für Bauelemente der organischen Elektronik, Molekularelektronik und organischen Optoelektronik (einschließlich der Herstellung organischer, Licht emittierender Dioden, sogenannter OLEDs).

Darüber hinaus umfasst die vorliegende Erfindung die Verwendung eines Materialsystems zur Erzeugung von Singulett-Sauerstoff aus wenigstens zwei Molekülen, wobei wenigstens ein Molekül eine Endoperoxid bildende Verbindung ist.

Ferner wäre es denkbar, die bisher erwähnten Nanopartikel des Materialsystems, einschließlich der mit Endoperoxid dotierten Liposomen, nach bekannten Verfahren auch an Antikörper zu binden und für therapeutische Zwecke anzuwenden.

Zudem ist auch denkbar, bei allen Anwendungen (z. B. technisch oder medizinisch) neben einem thermisch aktivierten Endoperoxidzerfall, den Prozess beispielsweise durch Infrarotlicht zu induzieren.

Im Allgemeinen kann das Materialsystem beispielsweise aus pharmazeutischen Trägermaterialien bestehen, welche voneinander getrennt Sensitizer-Moleküle und Endoperoxid bildenden Moleküle enthalten oder beispielsweise aus Trägermaterialien bestehen, in das nur die Endoperoxid bildenden Moleküle eingebaut sind, während sich der Sensitizer beispielsweise getrennt, also außerhalb des Trägermaterials, in Lösung befindet. Ferner kann das Materialsystem aus einem Trägermaterial bestehen, welches Multichromophormoleküle, bestehend aus einem Sensitizermolekül und min. 1 Endoperoxid bildendem Molekül, enthält, welche bevorzugt eine besondere Art der Autoperoxidation zeigen.

Weitere vorteilhafte Ausführungsformen ergeben sich aus den beigefügten Zeichnungen.

Darin zeigen:
- Fig. 1: eine schematische Darstellung der Thermolyse eines Endoperoxid bildenden Moleküls (1,4-Dimethylnaphtalin (DMN));
- Fig. 2: eine schematisches Kalottenmodell des DMN Endoperoxidmoleküls;
- Fig. 3: weitere funktionalisierte Endoperoxid bildende Moleküle;
- Fig. 4a: in einem Diagramm eine schematisch dargestellte Zerfallskurve; und
- Fig. 4b: in einem Diagramm eine veränderte schematisch dargestellte Zerfallskurve.

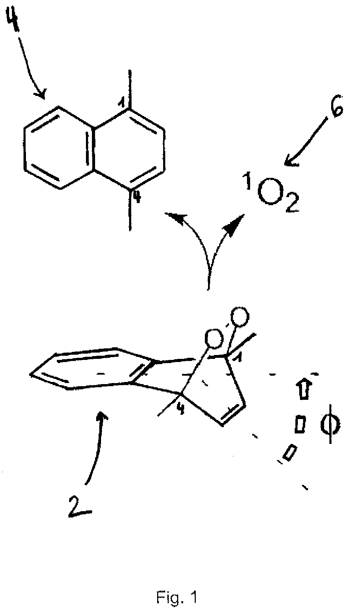

Fig. 1 zeigt eine schematische Darstellung der Thermolyse eines Endoperoxid bildenden Moleküls. Das Endoperoxidmolekül 2, hier 1,4-Dimethylnaphthalinendoperoxid, weist eine nach oben gerichtete Endoperoxidbrücke an Position 1 und 4 des Aromaten auf. Neben der vernachlässigbaren kleinen Toxizität von DMN, ist ein weiterer wichtiger Grund zur Verwendung von DMN-Endoperoxid 2, dass neben der genauen Kenntnis der Singulett-Sauerstofferzeugung beim Zerfall auch die räumliche Struktur sehr gut bekannt ist (siehe Wasserman et al. J. Parr. J. Org. Chem. 70 (2005) 105-109). Während des Prozesses der Singulett-Sauerstoff-Freisetzung zerfällt das nicht planare DMN-Endoperoxidmolekül 2, bei dem ein Ring um den Winkel Φ abgeknickt ist, unter Abspaltung von Singulett-Sauerstoff 6 wieder in das ursprüngliche aromatische und daher planare Molekül DMN 4, wobei der Knickwinkel Φ wieder zurück geklappt werden muss.

Die Energiebarriere für diesen Zerfallsprozess wird von der Wechselwirkung des Endoperoxidmoleküls 2 mit der Matrix 8 des Trägermaterials (Fig. 2a) beeinflusst. Dies bedeutet, dass das DMN-Endoperoxidmolekül 2 und die unmittelbare Nachbarschaft der umgebenden Trägermatrix 8 - gewissermaßen als spezielle Einbaulage - eine (supramolekulare) Funktionsstruktur bilden. Verwendet man als Trägermaterial Liposomen (die als flüssig-kristalliner Festkörper betrachtet werden können, so kann der Zerfall der in die Liposomenmembran eingebauten Endoperoxidmoleküle 2 durch chemische Veränderungen der Funktionsstruktur geändert werden. Beispielsweise kann durch das Einführen von funktionellen Gruppen (wie in Figur 3) ein Zurückklappen um den Knickwinkel Φ behindert (bzw. verzögert) werden.

Bei Polymeren dagegen spielen - wie bereits vorne beschrieben - auch physikalische Veränderungen der Funktionsstrukturen eine wichtige Rolle.

Fig. 2a zeigt das Endoperoxidmolekül 2 in einer Matrix 8 des Polymermaterials. Für einen Teil der Messungen zur Demonstration der Erhöhung der Stabilisierung, d. h. der Vergrößerung der Zerfallszeiten von Endoperoxiden durch physikalische Veränderungen der Funktionsstrukturen wurde u. a. das Polymer Polyvinylbutyral (PVB) verwendet. PVB ist ein Kopolymer mit verschiedenen Anteilen an Azetal-, Ester- und Hydroxylgruppen. Diese sind statistisch in der Polymerkette verteilt und behindern sich gegenseitig durch ihre unterschiedliche räumliche Struktur, so dass PVB-Schichten bevorzugt immer amorph vorliegen. Die meisten Seitengruppen sind polar und besitzen permanente Dipolmomente.

Das Polymer PVB wird hier bevorzugt in Form von Filmen und Nanopartikeln eingesetzt, während Ethylcellulose bevorzugt in Form von Nanopartikeln eingesetzt wird. Eine Beschreibung der Herstellung von Polymernanopartikeln findet man z. B. bei Sergey M. Borisov, et al, "Precipitation as a sample and versatile method for preperation of optical nanochemosensors", Talanta (2009) 1322-1330.

Raumgebiete mit freiem Volumen 12 in der Trägermatrix 8 sind bei der Herstellung der Polymerfilme oder der Polymernanopartikel (beispielsweise durch Ausfällung) wegen des Herausdiffundierens des Lösungsmittels entstanden. Je nach den lokalen geometrischen Verhältnissen entstehen in jeder Funktionsstruktur somit unterschiedlich große freie Volumina. Wenn das Endoperoxidmolekül 2 im Käfig der umgebenden Matrix 8 des Trägermaterials am rechten Ende des Rings 10 keine Bewegungsfreiheit hat, dann kann beispielsweise auch der linke Ring 11 hochgeklappt werden, wie in Fig. 2b dargestellt, um durch dieses Zurückklappen ebenfalls die ursprüngliche planare DMN-Struktur 4 wieder herzustellen.

Durch chemische Veränderungen, beispielsweise durch das Einführen von funktionellen Gruppen können zwischen Molekülen des Trägermaterials 8 und des Endoperoxidmoleküls 2 chemische Bindungen ausgebildet werden, welche ein Zurückklappen um den Knickwinkel Φ verzögern. Wie vorne bereits erwähnt sind neben den chemischen auch die physikalischen Veränderungen wichtig. Das Grundprinzip der physikalischen Veränderung einer speziellen Funktionsstruktur bestehend aus einem Endoperoxid bildenden Molekül und dem Käfig der umgebenden Polymermatrix beinhaltet folgendes Vorgehen bei der Präparation. Nach einer Prozedur der Endoperoxidbildung lässt man (bevorzugt thermisch aktiviert) einen Zerfallsprozess ablaufen, bei dem es durch Umlagerungsprozesse zu einer Veränderung der Funktionsstruktur kommt. Wird nach diesen Präparationsschritten erneut eine Endoperoxidbildung durchgeführt, so ergibt sich anschließend eine veränderte Zerfallszeit, welche auf die mit der geometrischen Änderung der Funktionsstruktur verknüpften Änderung der Barrierenhöhe für den Zerfall zurück zu führen ist.

In einem speziellen Präparationsverfahren wird mit Hilfe eines ersten Thermozyklus, also mit der Durchführung des Zerfallsprozesses für einen großen Teil der DMN-Endoperoxidmoleküle 2, welche in diesem Material 8 in zwei Einbaulagen mit unterschiedlicher Barrierehöhe vorhanden sind, vor dem anschließenden erneuten Endoperoxid-Bildungsprozess jeweils eine Barrierenerhöhung erzeugt. In diesem zur Präparation durchgeführten ersten thermisch aktivierten Zerfallsprozess werden durch den Zerfallsprozess starke Schwingungsanregungen der Moleküle (oder Molekülgruppen) in der Käfigwand 8 erzeugt und somit können Umlagerungsprozesse stattfinden, die Veränderungen der Funktionsstruktur bewirken. Diese Umlagerungsprozesse in der Käfigwand führen in PVB-Polymerfilmen zu einer Verminderung des freien Volumens 12 in der unmittelbaren Umgebung der Endoperoxid bildenden Moleküle und damit zur Erhöhung der Barrieren für den erneuten Zerfall (wegen der Behinderung beim Zurückklappen des Winkels Φ).

Zu erwähnen ist, dass hier nur der einfachste Fall von einem sich in einem freien Volumen 12 der Matrix 8 befindlichen Endoperoxidmolekül 2 gezeigt wurde. Anstatt diesem einen Endoperoxidmolekül ist es auch denkbar, dass beispielsweise funktionalisierte Molekülketten vorliegen, welche über Ester- oder Etherbindungen miteinander verbunden sind und mit den Molekülen der Matrix 8 des Trägermaterials in Wechselwirkung treten.

Fig. 3 zeigt weitere Endoperoxid bildende Moleküle 20 und 22, wobei 20 der 1,4,5-Trimethylnaphthalin (TMN) zeigt, bei welchem im Gegensatz zu 1,4-Dimethylnaphthalin am zweiten Ring eine weitere funktionelle Methylgruppe 21a angefügt ist. Diese weitere Methylgruppe stellt für das oben erwähnte Zurückklappen eine zusätzliche Behinderung dar, wenn die Methylgruppen frei rotieren können, so wie dies in der flüssig-kristallinen Phase der Fall ist, wobei das TMN-Endoperoxidmolekül zwischen den paraffinartigen Ketten der Membranmoleküle steckt. Diese zusätzliche sterische Behinderung erzeugt eine Erhöhung der Barriere für den Zerfall von TMN-Endoperoxid 20 (im Vergleich zu DMN-Endoperoxid in Liposomen) und damit eine Erhöhung der Halbwertszeit (auf ca. 17h bei 37°C). Die Halbwertszeit ist also damit bei TMN angepasst für eine Krebstherapie, da t_{z} deutlich größer ist als tₐ und es wurde eine Abtötung der Krebszellen für TMN-Endoperoxid, nicht aber für DMN-Endoperoxid in Liposomen, festgestellt. Dieser zytostatische Effekt wurde mittels Kristallviolett-Test bestätigt.

Ein weiteres Endoperoxid bildendes Molekül 22 weist eine zusätzliche Hydroxylgruppe auf, welche mit der Käfigwand Wasserstoff-Brücken (bevorzugt in PVB-Nanopartikeln, in PVB-Filmen oder Ethylcellulose-Nanopartikeln) ausbilden kann, was ebenfalls eine Erhöhung der Zerfallszeiten bedingt. Die Abtötung von Krebszellen wurde auch mit DMNOH-Endoperoxid in Ethylcellulose-Nanopartikeln erhalten.

Mit DMNOH in Ethylcellulose-Nanopartikeln konnte auch gezeigt werden, dass man die physikalische Veränderung der Funktionsstruktur auch in der Form durchführen kann, dass man den Zerfall nicht extra durch Temperaturerhöhung macht, sondern dass es genügt, den Prozess der Endoperoxid-Bildung extra langsam (15 Stunden) ablaufen zu lassen, so dass während dieser langen Zeit bereits bei niedrigerer Temperatur auch genügend Zerfallsprozesse stattfinden, um die Funktionsstrukturen entsprechend zu verändern.

In Fig. 4a ist der erste zeitliche Zerfall eines Endoperoxid bildenden Moleküls dargestellt, welches in einer Polymermatrix angeordnet ist.

Die Abszisse gibt die Zeit wieder, wohingegen an der Ordinate die Anzahl der Endoperoxidmoleküle logarithmisch aufgetragen ist.

Bei der Betrachtung der Zerfallskurven von DMN-Endoperoxiden in PVB-Matrix fällt auf, dass man - im Gegensatz zu dem monoexponentiellen Zerfall in allen Materialsystemen, bei denen ein Endoperoxid in Liposomen eingebaut ist - in guter Näherung einen biexponentiellen Zerfall hat. Es liegt zwar eine statistische Verteilung der Funktionsstrukturen vor, näherungsweise kann man aber in allen Polymersystemen von 2 Funktionsstrukturen ausgehen. Die gesamte Zerfallskurve wird üblicherweise auf dem Umweg über die Messung der Fluoreszenz der beim Zerfall der Endoperoxide wieder auftauchenden DMN-Moleküle ermittelt.

Bei halblogarithmischer Auftragung erhält man (bei 37 °C) die beiden Geraden 24 und 25 als Zerfallskurven. Bei der Auswertung ergibt sich, dass die Zerfallszeit genauer die Halbwertszeit des Zerfalls aus der Kurve 24 mit ca. 2 Stunden nur ca. 30 % größer ist, als die für DMN in organischen Lösungsmitteln (bei 37° C), während die Zerfallszeit aus der Kurve 25 bereits ca. einen Faktor 6 größer ist. Es ist naheliegend, der Kurve 24 eine Funktionsstruktur mit sehr großem freien Volumen zuzuordnen, also große Bewegungsfreiheit für das Zurückklappen um den Winkel Φ, während man der Kurve 25 zuzuordnenden Funktionsstrukturen ein geringeres freies Volumen zuordnen kann, so dass der Zerfall bereits verlangsamt abläuft. Wenn der Zerfall der Endoperoxide weitgehend abgeschlossen ist, hat man bereits das vorher beschriebene Vorgehen zur physikalischen Veränderung der Funktionsstrukturen durchgeführt. Nach erneuter Endoperoxidbildung erhält man bei 37 °C die Zerfallskurven 26 und 27, die jeweils nochmals deutlich flacher als Kurve 24 und 25 verlaufen; die Zerfallszeit von Kurve 27 ist um mehr als einen Faktor 30 länger als die von DMN in Flüssigkeiten, und der prozentuale Anteil der langsam zerfallenden Endoperoxide ist etwas größer geworden als bei Kurve 24 und 25.

Die physikalische Veränderung der Funktionsstrukturen bedeutet, dass die Gebiete mit (gro-βem) freien Volumen, die nach der ursprünglichen Probenpräparation direkt neben dem DMN-Molekül als Nichtgleichgewichtszustand vorhanden sind, mit Hilfe des Endoperoxidzerfalls verkleinert werden. Beim Zerfall kommt es zu Schwingungsanregungen, die zu Umlagerungsrozessen führen, bei denen sich die Moleküle der Käfigwand vor allem in Richtung des freien Volumens bewegen können. Dies führt zu einer Barrierenerhöhung und damit zu einer Verlängerung der Zerfallszeit für die veränderten Funktionsstrukturen.

Vergleichbare Ergebnisse wie mit Polymerfilmen erhält man mit DMN in Polymernanopartikeln aus PVB und aus Ethylcellulose. Bei Einsatz von DMNOH (siehe Fig. 3), das auf der Basis der von I. Saito et al (J. Am. Chem. Soc. 107 (1985) 6329-6334) angegebenen Synthese hergestellt wurde, konnte wegen der Veränderung der chemischen Funktionsstruktur durch Wasserstoffbrückenbindungen zwischen DMNOH-Molekülen und den OH-Gruppen der Ethylcellulose - ein um den Faktor 3 langsamerer Zerfall als in der Kurve 24 erhalten werden. Nach Durchführung der gleichen physikalischen Veränderungen der Funktionsstruktur (d. h. Endoperoxidzerfall) wurde nahezu der gleiche Wert wie bei DMN erhalten. Am Ende der neuen Zerfallskurve kann man nach einem dritten Schritt der Endoperoxidbildung nochmals den Zerfall messen und bekommt eine weitere Erhöhung der Zerfallszeit um ca. 50 %.

Die Anmelder behalten sich vor, sämtliche in den Anmeldungsunterlagen offenbarten Merkmale als erfindungswesentlich zu beanspruchen, sofern sie einzeln oder in Kombination gegenüber dem Stand der Technik neu sind.

### Bezugszeichenliste

- 2: Endoperoxidmolekül
- 4: Endoperoxid bildendes Molekül
- 6: Singulett-Sauerstoff
- 8: Matrix
- 10: rechter Ring des Endoperoxidmoleküls
- 11: linker Ring des Endoperoxidmoleküls
- 12: freies Volumen
- 20: TMN
- 21: Methylgruppe
- 21a: weitere Methylgruppe
- 22: DMNOH
- 23: Hydroxylgruppe
- 24: schneller Endoperoxidzerfall
- 25: langsamer Endoperoxidzerfall
- 26: veränderter schneller Endoperoxidzerfall
- 27: veränderter langsamer Endoperoxidzerfall

## Patentansprüche

1. Verfahren zur Herstellung von Singulett-Sauerstoff durch den Zerfall von Endoperoxidmolekülen,
wobei mit Hilfe eines sich aus wenigstens zwei Molekülen zusammensetzenden Materialsystems, das zumindest ein eine Endoperoxidgruppe bildendes Molekül enthält, eine Anpassung eines zeitlichen Verlaufs eines Zerfalls der Endoperoxidmoleküle ermöglicht wird,
wobei ein Molekül des Materialsystems ein Trägermaterial ist, wobei das Trägermaterial organisch ausgebildet ist und in seinem Volumen zahlreiche Moleküle aufweist und über eine geeignete mikroskopische und makroskopische Struktur verfügt, wobei die Endoperoxidmoleküle auf der Oberfläche des Trägermaterials angelagert oder in seinem Inneren eingebaut sind
**dadurch gekennzeichnet, dass**
die Anpassung des zeitlichen Verlaufs des Zerfalls der Endoperoxidmoleküle durch eine physikalische Veränderung der im Materialsystem vorhandenen Funktionsstrukturen erfolgt, wobei diese Funktionsstrukturen jeweils aus dem Endoperoxid bildenden Molekül und seiner unmittelbaren Umgebung im Trägermaterial nämlich einem Käfig der umgebenden Polymermatrix bestehen, wobei
die physikalische Veränderung einer Funktionsstruktur bei der Präparation das Ablaufenlassen eines Zerfallsprozess nach einer Prozedur der Endoperoxidbildung, bei dem es durch Umlagerungsprozesse zu einer Veränderung der Funktionsstruktur kommt, umfasst, wobei die physikalische Veränderung der Funktionsstruktur eine Veränderung der Einbaulage des Endoperoxid bildenden Moleküls im Käfig des umgebenden Trägermaterials und/oder eine Veränderung des Käfigs beziehungsweise der Käfigwand umfasst; und
nach diesem Präparationsschritt erneut eine Endoperoxidbildung durchgeführt wird, wobei sich eine veränderte Zerfallszeit ergibt, welche auf die mit der geometrischen Änderung der Funktionsstruktur verknüpfte Änderung der Barrierenhöhe für den Zerfall zurück zu führen ist.

2. Verfahren nach einem der vorangegangenen Ansprüche,
**dadurch gekennzeichnet, dass**
als Endoperoxid bildendes Molekül ein Naphthalinderivat, bevorzugt 1,4-Dimethylnaphthalin (DMN) oder eines dessen Derivate, eingesetzt wird.

3. Verfahren nach Anspruch 2
**dadurch gekennzeichnet, dass**
bei der physikalische Veränderung der Funktionsstrukturen die Gebiete mit freien Volumen, die nach der ursprünglichen Probenpräparation direkt neben dem DMN-Molekül als Nichtgleichgewichtszustand vorhanden sind, mit Hilfe des Endoperoxidzerfalls dadurch verkleinert werden, dass es beim Zerfall zu Schwingungsanregungen kommt, die zu Umlagerungsprozessen führen, bei denen sich die Moleküle der Käfigwand in Richtung des freien Volumens bewegen und eine Barrierenerhöhung und eine Verlängerung der Zerfallszeit für die veränderten Funktionsstrukturen herbeiführen.

4. Verfahren nach einem der vorangegangenen Ansprüche,
**dadurch gekennzeichnet, dass**
Raumgebiete mit freiem Volumen in der Trägermatrix bei der Herstellung der Polymerfilme oder der Polymernanopartikel durch das Herausdiffundierens des Lösungsmittels erzeugt werden.

5. Verfahren nach einem der vorangegangenen Ansprüche,
**dadurch gekennzeichnet, dass**
als Polymer Polyvinylbutyral (PVB) bevorzugt in Form von Filmen und Nanopartikeln eingesetzt wird und/oder Ethylcellulose in Form von Nanopartikeln eingesetzt wird

6. Verfahren nach einem der vorangegangenen Ansprüche,
**dadurch gekennzeichnet, dass**
die aromatische Verbindung durch Autoperoxidation Endoperoxide bildet.

7. Verfahren nach einem der vorangegangenen Ansprüche,
**dadurch gekennzeichnet, dass**
ein magnetisches Material im Materialsystem verwendet wird.

8. Verfahren nach einem der vorangegangenen Ansprüche,
**dadurch gekennzeichnet, dass**
ein Sensitizer im Materialsystem entfernbar eingesetzt wird.

9. Verfahren nach Anspruch 8,
**dadurch gekennzeichnet, dass**
der Sensitizer mit mindestens einem Endoperoxid bildenden Molekül über eine chemische Bindung in Verbindung steht.

10. Verfahren nach einem der Ansprüche 2 - 9,
**dadurch gekennzeichnet, dass**
bei der Singulett-Sauerstoff-Freisetzung das nicht planare DMN-Endoperoxidmolekül, bei dem ein Ring um den Winkel Φ abgeknickt ist, unter Abspaltung von Singulett-Sauerstoff in das aromatische und planare Molekül DMN um den Knickwinkel Φ wieder zurückgeklappt wird.

11. Verfahren nach einem der vorangegangenen Ansprüche,
**dadurch gekennzeichnet, dass**
es im pharmazeutischen und/oder medizinischen Bereich angewendet wird.

12. Verfahren nach einem der Ansprüche 2 - 11,
**dadurch gekennzeichnet, dass**
in einem Präparationsverfahren mit Hilfe eines ersten Thermozyklus, also mit der Durchführung des Zerfallsprozesses für einen großen Teil der DMN-Endoperoxidmoleküle, welche in diesem Material in zwei Einbaulagen mit unterschiedlicher Barrierehöhe vorhanden sind, vor dem anschließenden erneuten Endoperoxid-Bildungsprozess jeweils eine Barrierenerhöhung erzeugt wird.

13. Verfahren nach einem der vorangegangenen Ansprüche,
**dadurch gekennzeichnet, dass**
die Endoperoxid bildende Verbindung mit mindestens einem Molekül, welches benachbart angeordnet ist und nicht Teil der Endoperoxid bildenden Verbindung ist, eine chemische Bindung, bevorzugt mit dem Polymermaterial des Trägers, eingeht.

## Claims

1. Method for generating singlet oxygen by decomposition of endoperoxide molecules,
wherein by means of a material system comprising at least two molecules and containing at least one molecule forming an endoperoxide group, an adjustment of a temporal progression of a decomposition of the endoperoxide molecules is made possible,
wherein a molecule of the material system is a carrier material, wherein the carrier material is organic and has numerous molecules in its volume and provides a suitable microscopic and macroscopic structure, wherein the endoperoxide molecules are deposited on the surface of the carrier material or are incorporated in its interior,
**characterized in that**
adjusting of the temporal progression of the decomposition of the endoperoxide molecules is effected by a physical change in the functional structures present in the material system, wherein these functional structures each consisting of the endoperoxide-forming molecule and its immediate environment in the carrier material, namely a cage of the surrounding polymer matrix, wherein
the physical change of a functional structure during the preparation comprises conducting a decomposition process after a procedure of endoperoxide formation, in which a change of the functional structure is caused by rearrangement processes, wherein the physical change of the functional structure comprises a change of the assembly position of the endoperoxide-forming molecule in the cage of the surrounding carrier material and/or a change of the cage or the cage wall, respectively; and after this preparation step, endoperoxide formation is executed again, yielding in a modified decomposition time which is attributable to the change of the barrier height for the decomposition associated with the geometric change in the functional structure.

2. Method according to any one of the preceding claims,
**characterized in that**
a naphthalene derivative, preferably 1,4-dimethylnaphthalene (DMN) or one of its derivatives, is used as the endoperoxide-forming molecule.

3. Method according to claim 2,
**characterized in that**
during the physical change of the functional structures, the areas with free volume which, after the original sample preparation, are present directly next to the DMN molecule as a non-equilibrium state, are reduced by means of the endoperoxide decomposition **in that**, during the decomposition, vibrational excitations occur which lead to rearrangement processes in which the molecules of the cage wall move in the direction of the free volume and induce a heightening of the barrier and an increase of the decomposition time for the modified functional structures.

4. Method according to any one of the preceding claims,
**characterized in that**
compartment regions with free volume in the carrier matrix are created during the preparation by diffusion of the solvent out of the polymer films or the polymer nanoparticles.

5. Method according to any one of the preceding claims,
**characterized in that**
polyvinyl butyral (PVB) is used as the polymer preferably in the form of films and nanoparticles and/or ethyl cellulose is used in the form of nanoparticles.

6. Method according to any one of the preceding claims,
**characterized in that**
the aromatic compound forms endoperoxides by autoperoxidation.

7. Method according to any one of the preceding claims,
**characterized in that**
a magnetic material is used in the material system.

8. Method according to any one of the preceding claims,
**characterized in that**
a sensitizer is used removably in the material system.

9. Method according to claim 8,
**characterized in that**
the sensitizer is connected to at least one endoperoxide-forming molecule via a chemical bond.

10. Method according to any one of claims 2-9,
**characterized in that**
during singlet oxygen release the non-planar DMN endoperoxide molecule, in which a ring is kinked by the angle Φ, is folded back again by the kink angle Φ with cleavage of singlet oxygen into the aromatic and planar molecule DMN.

11. Method according to any one of the preceding claims,
**characterized in that**
it is used in the pharmaceutical and/or medical field.

12. Method according to one of the claims 2 - 11,
**characterized in that**
in a preparation process by means of a first thermal cycle, namely by conducting of the decomposition process for a large part of the DMN endoperoxide molecules, which are present in this material in two installation positions with different barrier heights, a barrier increase is generated in each case before the subsequent further endoperoxide formation process.

13. Method according to any of the preceding claims,
**characterized in that**
the endoperoxide-forming compound forms a chemical bond with at least one molecule, which is arranged adjacent and is not part of the endoperoxide-forming compound, preferably with the polymer material of the carrier.

## Revendications

1. Procédé de fabrication d'oxygène singulet par la désintégration de molécules d'endoperoxyde,
dans lequel une adaptation d'une évolution dans le temps d'une désintégration des molécules d'endoperoxyde est rendue possible à l'aide d'un système de matériaux se composant d'au moins deux molécules qui contient au moins une molécule formant un groupe endoperoxyde,
dans lequel une molécule du système de matériaux est un matériau de support, dans lequel le matériau de support est réalisé de manière organique et présente dans son volume de nombreuses molécules et dispose d'une structure microscopique et macroscopique adaptée, dans lequel les molécules d'endoperoxyde sont accumulées sur la surface du matériau de support ou sont installées à l'intérieur de celui-ci, **caractérisé en ce que**
l'adaptation de l'évolution dans le temps de la désintégration des molécules d'endoperoxyde est effectuée par une modification physique des structures fonctionnelles présentes dans le système de matériaux, dans lequel lesdites structures fonctionnelles sont constituées respectivement de la molécule formant l'endoperoxyde et de son environnement direct dans le matériau de support, à savoir d'un réseau de la matrice polymère qui l'entoure, dans lequel
la modification physique d'une structure fonctionnelle comprend lors de la préparation le fait de conduire le processus de désintégration après une procédure de l'obtention d'endoperoxyde, dans lequel des processus de transposition donnent lieu à une modification de la structure fonctionnelle, dans lequel la modification physique de la structure fonctionnelle comprend une modification de la position d'installation de la molécule formant l'endoperoxyde dans le réseau du matériau de support qui l'entoure et/ou une modification du réseau ou de la paroi de réseau ; et
une obtention d'endoperoxyde est mise en oeuvre une nouvelle fois après ladite étape de préparation, dans lequel il résulte un temps de désintégration modifié, lequel est à lier au changement, combiné au changement géométrique de la structure fonctionnelle, de la hauteur de barrière pour la désintégration.

2. Procédé selon l'une quelconque des revendications précédentes,
**caractérisé en ce que**
est employé en tant que molécule formant l'endoperoxyde un dérivé de naphtalène, de manière préférée du 1,4-diméthylnaphtalène (DMN) ou un de ses dérivés.

3. Procédé selon la revendication 2,
**caractérisé en ce que**
lors de la modification physique des structures fonctionnelles, les zones avec des volumes libres, qui sont présentes dans un état hors équilibre directement à côté de la molécule de DMN après la préparation d'échantillon initiale, sont réduites à l'aide de la désintégration d'endoperoxyde **en ce que** la désintégration donne lieu à des excitations vibratoires qui conduisent à des processus de transposition, dans lesquels les molécules de la paroi de réseau se déplacent en direction du volume libre et entraînent une élévation de barrière et un prolongement du temps de désintégration pour les structures fonctionnelles modifiées.

4. Procédé selon l'une quelconque des revendications précédentes,
**caractérisé en ce que**
des zones spatiales sont produites avec un volume libre dans la matrice de support lors de la fabrication de films de polymère ou des nanoparticules de polymère par diffusion du solvant.

5. Procédé selon l'une quelconque des revendications précédentes,
**caractérisé en ce que**
est employé en tant que polymère du polyvinyle de butyral (PVB) de manière préférée sous la forme de films et de nanoparticules et/ou de l'éthylcellulose sous la forme de nanoparticules.

6. Procédé selon l'une quelconque des revendications précédentes,
**caractérisé en ce que**
le composé aromatique forme par autoperoxydation des endoperoxydes.

7. Procédé selon l'une quelconque des revendications précédentes,
**caractérisé en ce que**
un matériau magnétique est utilisé dans le système de matériaux.

8. Procédé selon l'une quelconque des revendications précédentes,
**caractérisé en ce que**
un sensibilisateur est employé dans le système de matériaux de manière à pouvoir être éliminé.

9. Procédé selon la revendication 8,
**caractérisé en ce que**
le sensibilisateur est relié à au moins une molécule formant l'endoperoxyde par l'intermédiaire d'une liaison chimique.

10. Procédé selon l'une quelconque des revendications 2 - 9,
**caractérisé en ce que**
lors de la libération d'oxygène singulet, la molécule d'endoperoxyde de DMN non planaire, pour laquelle un composé cyclique est plié de l'angle Φ, est ramenée à nouveau selon l'angle de pliage Φ dans la molécule de DMN aromatique et planaire avec séparation de l'oxygène singulet.

11. Procédé selon l'une quelconque des revendications précédentes,
**caractérisé en ce que**
il est mis en application dans le domaine pharmaceutique et/ou médical.

12. Procédé selon l'une quelconque des revendications 2 - 11,
**caractérisé en ce que**
respectivement une élévation de barrière est produite avant le nouveau processus d'obtention d'endoperoxyde qui suit dans un procédé de préparation à l'aide d'un premier thermocycle, donc avec la mise en oeuvre du processus de désintégration pour une grande partie des molécules d'endoperoxyde à base de DMN, lesquelles sont présentes dans ce matériau dans deux positions d'installation avec des hauteurs de barrière différentes.

13. Procédé selon l'une quelconque des revendications précédentes,
**caractérisé en ce que**
la liaison formant l'endoperoxyde à au moins une molécule, laquelle est disposée de manière adjacente et ne fait pas partie de la liaison formant l'endoperoxyde, subit une liaison chimique, de manière préférée au matériau polymère du support.
